# EUROPEAN PATENT APPLICATION

(11) **EP 3 978 608 A1**
(43) Date of publication of application: **06.04.2022**
(21) Application number: 20200140.0
(22) Date of filing: 05.10.2020
(51) Int. Cl.: C12N 15/113

(54) **OLIGOMERIC COMPOUND FOR DYSTROPHIN RESCUE IN DMD PATIENTS THROUGHOUT SKIPPING OF EXON-51**

(71) Applicant: SQY Therapeutics, 78590 Noisy-le-Roi (FR); UNIVERSITE DE VERSAILLES-SAINT QUENTIN EN YVELINES, 78035 Versailles cedex (FR); Institut National de la Santé et de la Recherche Médicale, 75013 Paris (FR)
(72) Inventor: GARCIA, Luis, 78590 Noisy-Le-Roi (FR); GOYENVALLE, Aurélie, 78960 Voisins-Le-Bretonneux (FR); SVINARTCHOUK, Fedor, 94800 Villejuif (FR); GRIFFITH, Graziella, 78990 Elancourt (FR); AVRIL-DELPLANQUE, Aurélie, 78350 Jouy-En-Josas (FR)
(74) Representative: Brevalex

(57) **Abstract**

The present invention concerns an oligomeric compound comprising from 10 to 50 monomer subunits, at least part of the sequence of which is complementary to the following sequence: AAGGAAACUGCCAUCUCCAA (SEQ ID NO: 1 in the appended sequence listing). The present invention also concerns a pharmaceutical composition comprising said oligomeric compound and their use for treating Duchenne Muscular Dystrophy.

SEQUENCE LISTING

<110>  SQY-Therapeutics
       Université Versailles Saint-Quentin
       Inserm Transfert

<120>  OLIGOMERIC COMPOUND FOR DYSTROPHIN RESCUE IN DMD PATIENTS
       THROUGHOUT SKIPPING OF EXON-51

<130>  S71898 EP

<140>  EP20XXXXX
<141>  2020-10-05

<160>  9

<170>  PatentIn version 3.5

<210>  1
<211>  20
<212>  RNA
<213>  Homo sapiens


<220>
<221>  misc_feature
<222>  (1)..(20)
<223>  +45+64 of exon 51 of the pre mRNA encoded by the human DMD gene

<400>  1
aaggaaacug ccaucuccaa                                                  20


<210>  2
<211>  233
<212>  RNA
<213>  Homo sapiens


<220>
<221>  misc_feature
<222>  (1)..(233)
<223>  Exon 51 of the pre mRNA encoded by the human DMD gene

<400>  2
cuccuacuca gacuguuacu cuggugacac aaccuguggu uacuaaggaa acugccaucu      60

ccaaacuaga aaugccaucu uccuugaugu uggagguacc ugcucuggca gauuucaacc     120

gggcuuggac agaacuuacc gacuggcuuu cucugcuuga ucaaguuaua aaaucacaga     180

gggugauggu gggugaccuu gaggauauca acgagaugau caucaagcag aag            233


<210>  3
<211>  15
<212>  DNA
<213>  Unknown

<220>
<223>  Artificial sequence

<220>
<221>  misc_feature
<222>  (1)..(15)
<223>  Peptide REGONE (antisense oligonucleotide)

<220>
<221>  modified_base
<222>  (1)..(15)
<223>  All nucleosides are tricyclo-DNA nucleosides

<400>  3
ggagatggca gtttc                                                        15


<210>  4
<211>  15
<212>  DNA
<213>  Unknown

<220>
<223>  Artificial sequence


<220>
<221>  misc_feature
<222>  (1)..(15)
<223>  Peptide REGONE.7 (antisense oligonucleotide)

<220>
<221>  modified_base
<222>  (1)..(6)
<223>  Nucleosides 1-6 are tricyclo-DNA nucleosides

<220>
<221>  modified_base
<222>  (7)..(7)
<223>  Nucleoside 7 is a 2′-O-methyl RNA nucleoside

<220>
<221>  modified_base
<222>  (8)..(15)
<223>  Nucleosides 8-15 are tricyclo-DNA nucleosides

<400>  4
ggagatggca gtttc                                                        15


<210>  5
<211>  15
<212>  DNA
<213>  Unknown

<220>
<223>  Artificial sequence


<220>
<221>  misc_feature
<222>  (1)..(15)
<223>  Peptide REGONE.8 (antisense oligonucleotide)

<220>
<221>  modified_base

<222>  (1)..(7)
<223>  Nucleosides 1-7 are tricyclo-DNA nucleosides

<220>
<221>  modified_base
<222>  (8)..(8)
<223>  Nucleoside 8 is a 2'-O-methyl RNA nucleoside

<220>
<221>  modified_base
<222>  (9)..(15)
<223>  Nucleosides 9-15 are tricyclo-DNA nucleosides

<400>  5
ggagatggca gtttc                                                        15


<210>  6
<211>  15
<212>  DNA
<213>  Unknown

<220>
<223>  Artificial sequence


<220>
<221>  misc_feature
<222>  (1)..(15)
<223>  Peptide REGONE.9 (antisense oligonucleotide)

<220>
<221>  modified_base
<222>  (1)..(8)
<223>  Nucleosides 1-8 are tricyclo-DNA nucleosides

<220>
<221>  modified_base
<222>  (9)..(9)
<223>  Nucleoside 9 is a 2'-O-methyl RNA nucleoside

<220>
<221>  modified_base
<222>  (10)..(15)
<223>  Nucleosides 10-15 are tricyclo-DNA nucleosides

<400>  6
ggagatggca gtttc                                                        15


<210>  7
<211>  15
<212>  DNA
<213>  Unknown

<220>
<223>  Artificial sequence


<220>
<221>  misc_feature
<222>  (1)..(15)
<223>  Peptide REGONE.10 (antisense oligonucleotide)

<220>
<221>  modified_base
<222>  (1)..(9)
<223>  Nucleosides 1-9 are tricyclo-DNA nucleosides

<220>
<221>  modified_base
<222>  (10)..(10)
<223>  Nucleoside 10 is a 2'-O-methyl RNA nucleoside

<220>
<221>  modified_base
<222>  (11)..(15)
<223>  Nucleosides 11-15 are tricyclo-DNA nucleosides

<400>  7
ggagatggca gtttc                                                    15


<210>  8
<211>  15
<212>  DNA
<213>  Unknown

<220>
<223>  Artificial sequence


<220>
<221>  misc_feature
<222>  (1)..(15)
<223>  Oligomeric moiety of SYN51 (antisence oligonucleotide)

<220>
<221>  modified_base
<222>  (1)..(4)
<223>  Nucleosides 1-4 are tricyclo-DNA nucleosides

<220>
<221>  modified_base
<222>  (5)..(5)
<223>  Nucleoside 5 is a 2'-O-methyl RNA nucleoside

<220>
<221>  modified_base
<222>  (6)..(15)
<223>  Nucleosides 6-15 are tricyclo-DNA nucleosides

<400>  8
aagauggcat ttcta                                                    15


<210>  9
<211>  13
<212>  DNA
<213>  Unknown

<220>
<223>  Artificial sequence


<220>

<221>  misc_feature
<222>  (1)..(13)
<223>  Peptide M23D (antisense oligonucleotide)

<220>
<221>  modified_base
<222>  (1)..(13)
<223>  All nucleosides are tricyclo-DNA nucleosides

<400>  9
cctcggctta cct                                                          13

## Description

### TECHNICAL FIELD

The present invention belongs to the general technical field of therapeutic nucleic acid molecules and notably of therapeutic nucleic acid molecules useful for restoring dystrophin activity using splice-switching technology in patients with Duchenne muscular dystrophy (DMD).

More particularly, the present invention provides new oligomeric compounds possibly containing one or more tricyclo-deoxyribonucleic acid (tc-DNA) nucleosides and one or more lipid moieties covalently linked to said oligomeric compound either directly or via a spacer, for targeting the exon 51 of the human dystrophin gene.

These oligomeric compounds and, in particular, the one designed hereinafter as SQY51, meet the therapeutic needs of over ten percent of DMD patients with large deletions; they are compatible with systemic delivery, they access the whole musculature including heart and smooth muscles, they cross the blood brain barrier while displaying little bioaccumulation.

### PRIOR ART

The antisense technology, in particular Antisense Oligonucleotide (AON)-based approaches started about forty years ago after Zamecnik and Stephenson showed oligonucleotides could be used to downregulate the expression of specific genes. These AONs were originally unmodified synthetic DNA complementary to the targeted mRNA but it rapidly became evident that chemical modifications to protect from nuclease degradation were necessary, especially at the level of phosphodiester internucleotide linkages.

Phosphorothioate (PS) backbones are the most largely used chemical modifications to protect AONs from nuclease activity and increase their stability to target RNA. Typical PS differ from phosphodiester (PO) bonds by the non-bridging phosphate O-atoms being replaced with a S-atom which confers higher stability and increased cellular uptake. PS modifications have demonstrated an elevated efficacy due to an increased bioavailability compared to their PO counterparts and most of the drugs currently under clinical programs include PS bonds. Nevertheless, although their pharmacokinetic advantage, PS modified molecules are known to cause toxicity or undesirable effects mainly due to their capacity to bind plasma proteins. Acute reactions/effects of PS backbones may include immune cell activation, complement activation, that have been particularly reported in monkey studies or prolongation of clotting times that is known to be transient and normalize as oligonucleotides are cleared from blood. To note, low level but sustained complement activation may lead to depletion of complement and damage to the vascular system and kidney.

Another prime site for chemical modification is the 2'-position in the sugar moiety and it has been widely used in the antisense field (e.g., 2'-*O*-methyl (2'OMe), 2'-*O*-methoxyethyl (2'OMOE), 2'-fluorinated (2'F) and 2'-*O*-aminopropyl analogs). Many other structural modifications of the sugar backbone do exist, including phosphorodiamidate morpholino oligomer (PMO), peptide nucleic acid (PNA), locked nucleic acid (LNA), phosphoramidate and methyl-phophonate derivatives as well as tricyclo-DNAs (tc-DNAs). Such an arsenal of AONs has offered many therapeutic options comprising the manipulation of alternative splicing where the antisense molecules are so-called splice switching oligonucleotides (SSO). Here, antisense molecules are used to modulate the ratio of splicing variants or correct splicing defects by inducing exon inclusion or exon skipping; an approach which is suited for the treatment of many neuromuscular disorders including the Duchenne Muscular Dystrophy (DMD).

Duchenne muscular dystrophy (DMD) is an X-linked recessive disorder that affects one in every 3500 live male births. This disorder has an estimated prevalence amongst males of 1-9/100,000 in France, thus qualifying as orphan disease. It is caused by mutations in the *DMD* gene, which encodes dystrophin, a large protein of 427 kDa found in a variety of tissues, especially in muscle fibers (i.e. striated and smooth muscles) and neurons in particular regions of the central nervous system. Dystrophin is located close to the inner surface of the plasma membrane, connecting the actin cytoskeleton to the extracellular matrix through a membrane dystrophin-associated glycoprotein complex. Lack of dystrophin makes that skeletal muscle fibers are particularly vulnerable to mechanical stress and undergo recurrent cycles of necrosis. As a result, patients display progressive weakness of skeletal muscles, which are with time replaced by adipofibrotic tissue, leading to loss of ambulation by the age of twelve, whereupon premature death is caused by either respiratory failure or cardiomyopathy. In addition, about one third of DMD patients also display cognitive impairment suggesting a noteworthy disruption of neuronal and brain function.

The full-length dystrophin, translated from a major 14-kb mRNA transcript made of 79 exons, is a modular protein that can fortunately support the deletion of multiple exons provided the open reading frame is preserved. This phenomenon occurs in the clinically milder disease Becker Muscular Dystrophy (BMD), where deletions that maintain the open reading frame lead to the synthesis of truncated semi-functional forms of dystrophin. Although DMD is caused by a variety of types of mutations that occur across the gene, most of mutations are large deletions resulting in out-of-frame shortened mRNAs translated into unstable and nonfunctional truncated dystrophins. Hence, it was proposed, twenty years ago, that interfering with the splicing process of elected exons using AONs might be a suitable therapeutic approach for DMD to restore a semi-functional dystrophin thus converting severe DMD into milder BMD.

Two types of compounds have been extensively tested for antisense-induced exon skipping, the 2'-*O*-methyl-modified ribose oligomers with a full-length phosphorothioate backbone (2'OMe-PS) and the phosphorodiamidate morpholino oligomers (PMO). Both types of antisense molecules have been shown to rescue dystrophin in skeletal muscle after systemic delivery in animal models of DMD and in clinical trials. However, further studies using 2'OMe-PS and PMO AONs targeting exon 51 in DMD patients have failed to show marked clinical benefit, likely due to insufficient levels of dystrophin rescue **[1].**

The International application WO 2010/115993 proposes tricyclo-DNA antisense oligonucleotides (tc-DNA AONs) in which all nucleotides are modified by the introduction of a cyclopropane ring in order to restrict conformational flexibility of the backbone **[2].** These tc-DNA AONs can be designed for skipping a mutated exon 23 or a mutated exon 51 within a dystrophin pre-mRNA. The tc-DNA AONs designed for skipping a mutated exon 51 are tc-DNA AON H51 (+68+82), tc-DNA AON H51 (+70+84) and tc-DNA AON H51 (+73+87) with the numerical values referring to exon 51 of the human dystrophin gene (*DMD* gene). Moreover, the International application WO 2013/053928 discloses nucleic acid molecule containing a sequence of tricyclo nucleosides joined by internucleoside phosphorothioate linkages thus forming tricyclo-phosphorothioate DNA molecules (tc-DNA-PS) **[3].** This application illustrates the use of tc-DNA-PS antisense oligonucleotides for exon 23 skipping of dystrophin pre-mRNA, consolidated by further work in the mdx mouse model of DMD showing that tc-DNA AONs with full PS backbone induced effective skipping of exon 23 to levels 5-6-fold higher than that achieved with 2'OMe-PS and PMO corresponding AONs **[4].** This translated into a greater rescue of dystrophin protein levels, particularly in the diaphragm and heart, where levels reached 50% and 40% respectively, compared to wild-type mice after 12 weeks of treatment. However, the substitution of oxygen by sulfur in the phosphate ester backbone, while significantly improving biodistribution, promotes unspecific protein binding as well as activation of the innate immune system (e.g., complement activation, clotting and elevated proinflammatory cytokines), which may possibly result in the worst in acute toxicity, at best in long-term toxicity **[5,6].** Alternative schemes, such as controlling phosphorothioate stereochemistry, have been proposed to deal with this situation **[7],** but recent clinical testing in DMD patients have turned out unsatisfactory.

In addition, the International application WO 2018/193428 proposed another strategy consisting in combining an oligomeric compound comprising one or more tc-DNA nucleosides with one or more lipid moieties covalently linked to this oligomeric compound **[8].** Amongst the different oligomeric compounds to which at least one lipid moiety is combined, such as disclosed in this application, the compound SY-0487 has shown the best preliminary results in exon 51 skipping studies and thus is so far considered as the finest tc-DNA-based compound for skipping the exon-51 in DMD. The compound SY-0487 is designed as SYN51 in the present document.

The present inventors have set themselves the aim of proposing alternative therapeutic nucleic acid molecules useful for splice-switching technology in patients with DMD. More particularly, the present inventors have set themselves the aim of proposing alternative therapeutic nucleic acid molecules which do not present the drawbacks of the prior art molecules such as toxicity and usable for restoring a semi-functional dystrophin.

### DESCRIPTION OF THE INVENTION

The present invention makes it possible to resolve the technical problems as defined previously and to attain the aim that the inventors have set themselves.

Indeed, the inventors have identified a particular sequence in the *DMD* gene and more particularly in the exon 51 of the pre-mRNA encoded by the human *DMD* gene, the reverse complements of which form interesting compounds for treating patients suffering from DMD.

As will be understood by those skilled in the art, in the cell nucleus, eukaryotic genes are transcribed into pre messenger RNAs (pre-mRNA), which contain both exons and introns. To form mature mRNA, splicing occurs at specific sequences at the borders of exons and introns (splice sites) thereby removing introns and connecting exons to one another to form mRNA, which is later translated into protein. During the three last decades, splice-switching approaches have been developed to interfere with such mechanisms in view of DMD treatment. In particular, successful skipping of exon 23 of the mouse *DMD* gene has been reported in mdx mice by using various types of antisense oligonucleotides annealing the donor splice site at the 3' end of exon 23. For exon 51, those skilled in the art have developed a number of compounds, some already evaluated at clinical level, selected after screening studies on patient cells, most of them targeting sequences comprised in the region spanning from +66+95 of exon 51 of the pre-mRNA encoded by the human *DMD* gene.

In the present invention, the particular sequences that were selected are located upstream of the region usually targeted by those skilled in the art; likely because the region covering the sequences implemented in the invention did not appear to be particularly outstanding during *in vitro* screens as practiced by those skilled in the art. The expression *"in vitro"* refers to an event that takes places outside of a subject's body. *In vitro* assays encompass cell-based assays in which cells alive or dead are employed and may also encompass a cell-free assay in which no intact cells are employed. On the contrary, the expression *"in vivo"* refers to an event that takes place in a subject's body.

It has been surprisingly found that the oligomeric compounds of the present invention, in particular when covalently linked to a lipid moiety such as a palmitoyl, has unexpected binding properties for serum proteins. Whatever the species considered, such class of compounds typically binds apolipoproteins (i.e., structural protein components of HDL and LDL), whereas the compounds of the invention preferentially and favorably bind serum albumin in human and non-human primate blood samples; such property potentially markedly improving the bioavailability of the compound as well as its dissemination in skeletal muscles and cardiac tissue. It is noteworthy that this valuable advantage is specific of the oligomeric compounds of the invention.

Thus the present invention concerns an oligomeric compound comprising from 10 to 50 monomer subunits, at least part of the sequence of which is complementary to the following sequence: AAGGAAACUGCCAUCUCCAA (SEQ ID NO: 1 in the appended sequence listing).

The expression "oligomeric compound" and the term "oligonucleotide" refer to a synthetic compound comprising from 10 to 50 monomer subunits linked by internucleosidic linkage groups. The length of an oligonucleotide may be denoted by the number of monomer subunits concatenated to the term "-mer". For example, an oligonucleotide containing ten monomer subunits is a 10-mer (or decamer), and an oligonucleotide containing 25 monomer subunits is a 25-mer. Oligonucleotides and oligomeric compounds of the present disclosure are listed from left to right following the order of the 5' to the 3' end, respectively.

The expression "monomer subunits", as used herein, is meant to include all manner of monomer units that are amenable to oligomer synthesis including, and typically referring to monomer subunits such as α-D-ribonucleosides, β-D-ribonucleosides, α-D-2'-deoxyribonucleosides, β-D-2'-deoxyribonucleosides, naturally occurring nucleosides, modified nucleosides, and hereby in particular tricyclo-deoxyribonucleic acid (tc-DNA) nucleosides, 2'-modified ribonucleic acid (2'-modified-RNA) nucleosides, locked nucleic acid (LNA) nucleosides, peptide nucleic acids (PNAs) nucleosides, 2'-deoxy 2'-fluoro-arabino nucleosides, hexitol nucleic acids (HNAs) nucleosides; and phosphorodiamidate morpholino (PMO) nucleosides, mimetics of nucleosides, naturally occurring nucleotides, modified nucleotides, and hereby in particular tricyclo-deoxyribonucleic acid (tc-DNA) nucleotides and 2'-modified ribonucleic acid (2'-modified-RNA) nucleotides, and mimetics of nucleotides. Advantageously, the expression "monomer subunit", as used herein, refers to naturally occurring nucleosides and modified nucleosides, and hereby in particular to ribonucleosides, deoxyribonucleosides, tricyclo-deoxyribonucleic acid (tc-DNA) nucleosides, 2'-modified ribonucleic acid (2'-modified-RNA) nucleosides, locked nucleic acid (LNA) nucleosides, peptide nucleic acids (PNAs) nucleosides, 2'-deoxy-2'-fluoro-arabinonucleosides, hexitol nucleic acids (HNAs) nucleosides and phosphorodiamidate morpholino (PMO) nucleosides, and to naturally occurring nucleotides and modified nucleotides, and hereby in particular to ribonucleotides, deoxyribonucleotides, tricyclo-deoxyribonucleic acid (tc-DNA) nucleotides, 2'-modified ribonucleic acid (2'-modified-RNA) nucleotides, locked nucleic acid (LNA) nucleotides, peptide nucleic acids (PNAs) nucleotides, 2'-deoxy-2'-fluoro-arabinonucleotides, hexitol nucleic acids (HNAs) nucleotides and phosphorodiamidate morpholino (PMO) nucleotides. More particularly, the expression "monomer subunit", as used herein, refers to modified nucleotides, and hereby in particular tricyclo-deoxyribonucleic acid (tc-DNA) nucleotides and 2'-modified ribonucleic acid (2'-modified-RNA) nucleotides.

Advantageously and depending on the monomer subunits they comprise, the oligomeric compounds of the present invention comprise or consist of oligodeoxyribonucleotides, oligoribonucleotides, morpholinos, tricyclo-DNA oligonucleotides, tricyclo-phosphorothioate-DNA oligonucleotides and LNA oligonucleotides.

It should be noted that in the monomer subunits comprised in the oligomeric compound according to the present invention, one can find not only the five classical nucleobases i.e. adenine (A), cytosine (C), guanine (G), thymine (T), and uracil (U) but also base analogs.

The term "base analog", also referred to as "modified nucleobase", refers to chemical modifications of DNA or RNA bases with a molecular structure that mimics natural DNA or RNA bases. Base analogs include, but are not limited to, 5-methylcytosine, 5-bromouracil, inosine, 5-substituted pyrimidines, 6-azapyrimidines and N-2, N-6 and O-6 substituted purines, including 2-aminopropyladenine, 5-propynyluracil and 5-propynylcytosine. Base analogs also include, but are not limited to, 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine, 2-thiocytosine, 5-halouracil and cytosine, 5-propinyluracil and 5-propinylcytosine (and other alkynyl derivatives of pyrimidine bases), 6-azouracil, 6-azocytosine, 6-azothymine, 5-uracil (pseudouracil), 4-thiouracil, 8-halo, 8-amino, 8-thiol, 8-thioalkyl, 8-hydroxyl and other 8-substituted adenines and guanines, 5-halo and particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deaza-adenine, 3-deazaguanine and 3-deaza-adenine, universal bases, tricyclic pyrimidines such as phenoxazine cytidine(1*H-*pyrimido [5,46][I,4]benzoxazin-2(3*H*)-one), phenothiazine cytidine (1*H*-pyrimido[5,4-6][ 1,4]benzothiazine-2(3*H*)-one), G-clamps such as a substituted phenoxazine cytidine (e.g. 9-(2-aminoethoxy)-*H*-pyrimido[5,4-6][1,4]benzoxazin-2(3*H*)-one), carbazole cytidine (2*H-*pyrimido[4,5-b]indol-2-one), and pyridoindole cytidine (2*H*-pyrido[3',2':4,5]pyrrolo[2,3-<f]pyrimidin-2-one). Base analogs may also include those in which the purine or pyrimidine base is replaced with other heterocycles, for example 7-deaza-adenine, 7-deazaguanosine, 2-aminopyridine and 2-pyridone. The preparation of modified nucleobases is known in the art.

The sequence AAGGAAACUGCCAUCUCCAA (SEQ ID NO: 1 in the appended sequence listing) to which part of the sequence of the oligomeric compound according to the present invention is complementary is the region defined by positions +45+64 of exon 51 of the pre-mRNA encoded by the human *DMD* gene. The exon 51 of the pre-mRNA encoded by the human *DMD* gene is of sequence:
CUCCUACUCAGACUGUUACUCUGGUGACACAACCUGUGGUUACUAAGGA AACUGCCAUCUCCAAACUAGAAAUGCCAUCUUCCUUGAUGUUGGAGGUACCUGCUCUGGCA GAUUUCAACCGGGCUUGGACAGAACUUACCGACUGGCUUUCUCUGCUUGAUCAAGUUAUAA AAUCACAGAGGGUGAUGGUGGGUGACCUUGAGGAUAUCAACGAGAUGAUCAUCAAGCAGA AG (SEQ ID NO: 2 in the appended sequence listing).

Advantageously, at least part of the sequence of the oligomeric compound according to the present invention is complementary to the sequence corresponding to the region +48+62 of SEQ ID NO: 2 in the appended sequence listing. This region also corresponds to the region +4+18 of SEQ ID NO: 1 in the appended sequence listing.

The term "complementary", as used herein, refers to a nucleic acid sequence that can form hydrogen bond(s) with another nucleic acid sequence by either traditional Watson Crick base pairing or other non-traditional types of pairing (e.g., Hoogsteen or reversed Hoogsteen hydrogen bonding) between complementary nucleosides or nucleotides. "Complementary" (or "specifically hybridizable") are terms that indicate a sufficient degree of complementarity or precise pairing such that stable and specific binding occurs between an oligomeric compound and a pre-mRNA or mRNA target.

It is understood in the art that a nucleic acid molecule need not be 100% complementary to a target nucleic acid sequence to be complementary. That is, two nucleic acid molecules may be less than fully complementary. Complementarity is indicated by a percentage of contiguous residues in a nucleic acid molecule that can form hydrogen bonds with a second nucleic acid molecule. For example, if a first nucleic acid molecule has 10 nucleotides and a second nucleic acid molecule has 10 nucleotides, then base pairing of 5, 6, 7, 8, 9, or 10 nucleotides between the first and second nucleic acid molecules represents 50%, 60%, 70%, 80%, 90%, and 100% complementarity, respectively. "Fully" complementary nucleic acid molecules means those in which all the contiguous residues of a first nucleic acid molecule form hydrogen bonds with the same number of contiguous residues in a second nucleic acid molecule, wherein the nucleic acid molecules either both have the same number of nucleotides (i.e., have the same length) or the two molecules have different lengths.

Thus, the oligomeric compound of the present invention and the target nucleotide sequence of the pre-mRNA are complementary to each other when a sufficient number of corresponding positions in each molecule are occupied by nucleobases that can bond with each other to allow stable association between the oligomeric compound of the present invention and the target nucleotide sequence of the pre-mRNA as indicated above. One skilled in the art recognizes that the inclusion of mismatches is possible without eliminating the ability of the oligomeric compounds to remain in association. Therefore, described herein are oligomeric compounds of the present invention, advantageously being antisense oligonucleotides, that may comprise up to about 20% nucleotides that are mismatched (i.e., are not nucleobase complementary to the corresponding nucleotides of the target). In particular the oligomeric compounds of the present invention, advantageously being antisense oligonucleotides, contain no more than about 15%, more preferably not more than about 10%, most preferably not more than 5% or no mismatches.

Typically, the oligomeric compound according to the present invention comprises or consists of an antisense oligonucleotide.

The expression "antisense oligonucleotide" refers to a single strand of DNA or RNA or oligomeric compound that is complementary to a targeted sequence. An antisense oligonucleotide is capable of hybridizing to a pre-mRNA or a mRNA having a complementary coding or non-coding nucleotide sequence. In the present case, this targeted nucleotide sequence corresponds to sequence SEQ ID NO: 1 in the appended sequence listing and typically to the sequence corresponding to the region +48+62 of SEQ ID NO: 2 in the appended sequence listing.

In addition, in the present invention, the antisense oligonucleotide (AON) sequences are selected so as to be specific, i.e. the AON's are fully complementary only to the sequences of the targeted pre-mRNA and not to other nucleic acid sequences. The AONs used in the practice of the invention may be of any suitable type (e.g., oligodeoxyribonucleotides, oligoribonucleotides, morpholinos, tricyclo-DNA, tricyclo-phosphorothioate-DNA, LNA, U7- or U1-modified AONs or conjugate products thereof such as peptide-conjugated or nanoparticle-complexed AONs), which are known to the skilled person in the art **[9].** Oligomeric compounds and in particular AONs according to the invention are generally from about 10 to about 50 nucleotides in length, in particular from about 11 to about 40 nucleotides, from about 12 to about 30 nucleotides or from about 13 to about 20 nucleotides, and may be for example, about 10, or about 15, or about 20 or about 30 nucleotides or more in length. Typically, morpholino-AONs are about 25-30 nucleotides long, PPMO AONs are about 20-25 nucleotides long, and tricyclo-AONs are about 10-20 nucleotides long, U7 and U1-modified AONs may possibly carry longer antisense sequences of about 50 nucleotides. The expression "about X nucleotides" means X nucleotides ± 2 nucleotides.

In a particular embodiment, the oligomeric compound according to the present invention comprises at least one nucleotide sequence having at least 70% identity with the reverse complement of SEQ ID NO: 1.

Thus, the oligomeric compound according to the present invention comprises at least one nucleotide sequence having at least 70% identity and may exhibit at least 75%, at least 80%, at least 85%, at least 90% or even at least 95% identity with the reverse complement of SEQ ID NO: 1.

In a more particular embodiment, the oligomeric compound according to the present invention comprises at least one nucleotide sequence having at least 70% identity with the following tc-DNA nucleotide sequence:
GGAGATGGCAGTTTC (SEQ ID NO: 3 in the appended sequence listing).

Thus, the oligomeric compound according to the present invention comprises at least one nucleotide sequence having at least 70% identity and may exhibit at least 73%, at least 75%, at least 80%, at least 85%, at least 90% or even at least 93% identity with the tc-DNA nucleotide sequence SEQ ID NO: 3.

By "identity percent" between two nucleotide sequences (or between two amino acid sequences), it is meant, within the scope of the present invention, a percent of identical nucleotide (or amino acid) residues between the two sequences being compared, this percent being obtained after implementing the best alignment (optimum alignment) between both sequences. Those skilled in the art know different techniques enabling such an identity percent to be obtained and involving homology algorithms or computer programs such as the program BLAST.

The identity percent is statistic and the differences between both sequences are randomly distributed along these sequences. The differences between both sequences can consist of different modification types of the sequences: deletions, substitutions or additions of nucleotide (or amino acid) residues.

In a particular embodiment, the oligomeric compound according to the present invention comprises at least one nucleotide sequence identical to the tc-DNA nucleotide sequence SEQ ID NO: 3.

For use *in vivo,* the oligomeric compounds and in particular AONs according to the invention may be stabilized. A "stabilized" oligomeric compound or AON refers to an oligomeric compound or AON that is relatively resistant to *in vivo* degradation (e.g., via an exo- or endo-nuclease). Stabilization can be a function of length or secondary structure. Alternatively, oligomeric compound or AON stabilization can be accomplished via phosphate backbone modifications.

Preferred stabilized oligomeric compounds or AONs of the instant invention have a modified backbone, e.g., have phosphorothioate linkages to provide maximal activity and protect the oligomeric compound or AON from degradation by intracellular exo- and endo-nucleases. Other possible stabilizing modifications include phosphodiester modifications, combinations of phosphodiester and phosphorothioate modifications, methyl-phosphonate, methyl-phosphorothioate, phosphorodithioate, p-ethoxy, and combinations thereof. Chemically stabilized, modified versions of the oligomeric compounds or AONs also include "Morpholinos" (phosphorodiamidate morpholino oligomers, PMOs), 2'-*O*-Met oligomers, tricyclo-DNA (tc-DNA) oligomers **[2],** tricyclo-phosphorothioate DNA oligomers **[3],** LNAs etc., which are all known to the skilled person in the art **[9].**

The term "tricyclo-DNA" (tc-DNA) refers to a class of constrained oligodeoxyribonucleotide analogs in which each nucleotide is modified by the introduction of a cyclopropane ring to restrict conformational flexibility of the backbone and to optimize the backbone geometry of the torsion angle Y. In detail, the tc-DNA differs structurally from DNA by an additional ethylene bridge between the centers C(3') and C(5') of the nucleosides, to which a cyclopropane unit is fused for further enhancement of structural rigidity.

In a particular embodiment of the present invention, the oligomeric compound comprises mainly tricyclo-deoxyribonucleic acid (tc-DNA) nucleosides. Consequently, the oligomeric compound of the invention comprises or consists of a tricyclo-DNA antisense oligonucleotide. In this embodiment, the tricyclo-DNA antisense oligonucleotide according to the present invention comprises or consists of a nucleotide sequence corresponding to the nucleotide sequence SEQ ID NO: 3.

In this embodiment, the different tc-DNA nucleosides may be joined by phosphodiester linkages. Alternatively, at least two adjacent tc-DNA nucleosides may be joined by a phosphorothioate (PS) linkage. The expressions "phosphorothioate linkage" or "phosphorothioate modification", as interchangeably used herein, refers to a "5'... -OP(S)-O- ... 3" moiety between two adjacent nucleosides in a nucleic acid molecule. Advantageously all the tc-DNA nucleosides in the oligomeric compound according to the invention are joined by PS linkages. Thus the oligomeric compound of the invention comprises or consists of a tricyclo-phosphorothiate DNA antisense oligonucleotide. If other modifications are present in the oligomeric compound of the invention, the latter include phosphodiester, methylphosphonate, methyl-phosphorothioate, phosphorodithioate, and p-ethoxy modifications, and combinations thereof.

In another particular embodiment of the present invention, the oligomeric compound comprises at least one tricyclo-deoxyribonucleic acid (tc-DNA) nucleoside and at least one modified ribonucleic acid nucleoside. Any modified RNA nucleoside known to those skilled in the art can be implemented in the present invention. Modified RNA nucleosides confer flexibility to the oligomeric molecule in which they are introduced. In particular, this modified RNA nucleoside is a 2'-modified RNA nucleoside such as 2'-*O*-methyl, 2'-methoxyethoxy, 2'-fluoro, 2'-allyl, 2'-*O*-[2-(methylamino)-2-oxoethyl], 2'-amino and 2'-*O*-(N-methylcarbamate). More particularly, this modified RNA nucleoside is a 2'-*O*-methyl RNA nucleoside. In addition, the monomer subunits of the oligomeric compound according to this particular embodiment are typically joined by phosphodiester internucleoside linkages.

Advantageously, in this particular embodiment, the oligomeric compound can comprise several tricyclo-deoxyribonucleic acid (tc-DNA) nucleosides and at least one modified ribonucleic acid nucleoside and advantageously only one modified ribonucleic acid nucleoside. The latter can be present anywhere in the sequence of the oligomeric compound according to the present invention.

In particular, the oligomeric compound according to the present invention comprises or consists of a nucleotide sequence corresponding to one of the following nucleotide sequences:
- 5'-GGAGAT**g**GCAGTTTC-3' (SEQ ID NO: 4 in the appended sequence listing),
- 5'-GGAGATG**g**CAGTTTC-3' (SEQ ID NO: 5 in the appended sequence listing),
- 5'-GGAGATGG**c**AGTTTC-3' (SEQ ID NO: 6 in the appended sequence listing), and
- 5'-GGAGATGGC**a**GTTTC-3' (SEQ ID NO: 7 in the appended sequence listing).
in which tcDNA nucleotides are typed in capital letters while the modified ribonucleic acid nucleoside as previously defined is typed in lowercase letter.

In particular, the modified RNA nucleoside at positions +7, +8, +9 and +10, respectively in the above sequences is a 2'-modified RNA nucleoside and, more particularly, a 2'-*O*-methyl RNA nucleoside. In this last alternative, when the oligomeric compound consists of a nucleotide sequence corresponding to the sequence SEQ ID NO: 4, SEQ ID NO: 5, SEQ ID NO: 6 or SEQ ID NO: 7, this compound is designed as REGONE.7, REGONE.8, REGONE.9 or REGONE.10 respectively.

In a particular embodiment, the oligomeric compound according to the present invention can be combined with one or more lipid moieties. In other words, one or more lipid moieties can be covalently linked to the oligomeric compound either directly or indirectly i.e. via a spacer.

The term "lipid moiety" as used herein refers to moieties that are derived from, typically and advantageously, hydrocarbons, oils, fats (such as fatty acids, glycerides), sterols, steroids, and derivative forms of these compounds. Suitable lipid moieties include moieties derived from fatty acids and their derivatives, hydrocarbons and their derivatives, and sterols, such as cholesterol. As used herein, the term lipid moiety also includes amphipathic compound moieties, which contain both lipid and hydrophilic moieties. The definitions given for all these different moieties in **[8]** also apply to the present invention.

Any lipid moiety disclosed in **[8]** can be implemented in the present invention. In a particular embodiment, at least one lipid moiety linked to the oligomeric compound is a saturated fatty acid moiety, more particularly a saturated fatty acid moiety derived from palmitic acid (C16) or stearic acid (C18) and most particularly a saturated fatty acid moiety derived from palmitic acid (C16).

In the present invention, at least one lipid moiety is linked to the oligomeric compound at (i) a terminal residue of said oligomeric compound, (ii) the 5'-terminus of said oligomeric compound, (iii) the 3'-terminus of said oligomeric compound; or (iv) an internal residue of said oligomeric compound.

When the linkage between the oligomeric compound and at least one lipid moiety is direct, the covalent link implies one atom of the oligomeric compound and one atom of the at least one lipid moiety.

When the linkage between the oligomeric compound and the at least one lipid moiety is indirect, there is a spacer. One first atom of this spacer is covalently linked to one atom of the oligomeric compound while a second atom of this spacer different from the first one is covalently linked to one atom of the at least one lipid moiety.

Any spacer disclosed in **[8]** can be implemented in the present invention. In a particular embodiment, the spacer implemented in the invention is of below formula (I) or (II):

ø-NH-C₂₋₁₂alkylene-OP(=O)(OH)-§ (I)

ø-NH-C₂₋₁₂alkylene-OP(=S)(OH)-§ (II),

in which (ø) represents the point of covalent linkage to the lipid moiety and (§) represents the point of covalent linkage to the oligomeric compound.

It should be noted that the spacer of formula (II) is in equilibrium with the spacer of below formula (II'):

ø-NH-C₂₋₁₂alkylene-OP(SH)(=0)-§ (II').

Thus formula (II) and formula (II') are equivalent and can be used interchangeably.

In a more particular embodiment, the spacer is of formula (II) and advantageously the alkylene chain this spacer is 6 carbon atoms long.

As particular examples of the oligomeric compound according to the present invention, one can cite:
- palmitate-NH-C₆alkylene-OP(=S)(OH)-REGONE.7,
- palmitate-NH-C₆alkylene-OP(=S)(OH)-REGONE.8 (hereinafter designed as SKY51),
- palmitate-NH-C₆alkylene-OP(=S)(OH)-REGONE.9, and
- palmitate-NH-C₆alkylene-OP(=S)(OH)-REGONE.10.

In other words, the oligomeric compound according to the present invention it is selected from the group consisting of:
- palmitate-NH-C₆alkylene-OP(=S)(OH)-GGAGAT**g**GCAGTTTC-3' (SEQ ID NO: 4 in the appended sequence listing),
- palmitate-NH-C₆alkylene-OP(=S)(OH)-GGAGATG**g**CAGTTTC-3' (SEQ ID NO: 5 in the appended sequence listing),
- palmitate-NH-C₆alkylene-OP(=S)(OH)-GGAGATGG**c**AGTTTC-3' (SEQ ID NO: 6 in the appended sequence listing), and
- palmitate-NH-C₆alkylene-OP(=S)(OH)-GGAGATGGC**a**GTTTC-3' (SEQ ID NO: 7 in the appended sequence listing).

The present invention concerns an oligomeric compound as previously defined for use as a medicament. Thus the present invention relates to a pharmaceutical composition comprising, as an active ingredient, an oligomeric compound according to the present invention and a pharmaceutically acceptable vehicle.

By "pharmaceutically acceptable vehicle", it is meant according to the present invention, any substance which is added to an oligomeric compound according to the present invention to promote its transport, avoid its substantial degradation in said composition and/or increase its half-life. Advantageously, such a pharmaceutically acceptable vehicle is sterile and nonpyrogenic and refers to molecular entities and compositions that do not produce an adverse, allergic or other untoward reaction when administered to a mammal, especially a human, as appropriate. It is chosen depending on the type of application of the pharmaceutical composition of the invention and in particular as a function of its administration mode. Advantageously, a pharmaceutically acceptable vehicle refers to a non-toxic solid, semi-solid or liquid filler, diluent, encapsulating material or formulation auxiliary of any type.

The pharmaceutical compositions according to the invention can be employed by the systemic route; by the parenteral route, for example the intravenous, intra-arterial, intraperitoneal, intrathecal, intra-ventricular, intrasternal, intracranial, intramuscular or sub-cutaneous route; by topical route; by the oral route; by the rectal route; by the intranasal route or by inhalation.

As solid compositions for oral administration, tablets, pills, powders, etc. can be used where the oligomeric compound according to the invention is mixed with one or more conventionally used inert diluents, and possibly other substances such as, for example, a lubricant, a colorant, a coating etc.

As liquid compositions for oral or ocular administration pharmaceutically acceptable, suspensions, solutions, emulsions, syrups containing conventionally used inert diluents, and possibly other substances such as wetting products, sweeteners, thickeners, etc. can be used.

The sterile compositions for parenteral administration can be aqueous or non-aqueous (oleaginous) solutions, suspensions or emulsions. As a solvent or vehicle, water, propylene-glycol, plant oils or other suitable organic solvents can be used. These compositions can also contain adjuvants, such as dispensing or wetting agents, suspending agents, isotonisers, emulsifiers, etc.

The compositions for topic administration can be for example creams, lotions, oral sprays, nose or eye drops or aerosol.

Those skilled in the art will recognize that the amount of an oligomeric compound to be administered will be an amount that is sufficient to induce amelioration of unwanted disease symptoms. Such an amount may vary inter alia depending on such factors as the age, weight, overall physical condition, of the patient, etc. and may be determined on a case by case basis. The amount may also vary according to the other components of a treatment protocol (e.g., administration of other medicaments such as steroids, etc.). Those skilled in the art will recognize that such parameters are normally worked out during clinical trials. Further, those skilled in the art will recognize that, while disease symptoms may be completely alleviated by the treatments described herein, this is not an absolute requirement. Even a partial or intermittent relief of symptoms may be of great benefit to the recipient. In addition, treatment of the patient is usually not a single event. Rather, the oligomeric compounds of the invention will likely be administered on multiple occasions, that may be, depending on the results obtained, several days apart, several weeks apart, or several months apart, or even several years apart.

The present invention also concerns an oligomeric compound as previously defined or a pharmaceutical composition as previously defined for use in treating Duchenne Muscular Dystrophy in a patient in need.

In the context of the invention, the term "patient" refers to any subject, afflicted with DMD disease and harboring a large genetic deletion provoking frameshift mutation in the gene coding dystrophin, which could be restored by removing exon 51 during mRNA splicing.

In its broadest meaning, the term "treating" or "treatment" refers to reversing, alleviating, inhibiting the progress of, or preventing the disorder or condition to which such term applies, or one or more symptoms of such disorder or condition.

The term "splicing" is known to the skilled person in the art, and used herein accordingly. The term "splicing", as used herein, refer to the modification of a pre-mRNA following transcription, in which introns are removed and exons are joined.

The expression "exon skipping", as used herein, refers to the process leading to the removal from the fully-processed mRNA of an exon which would have been otherwise left in the mature mRNA. By blocking access of spliceosome to one or more splice donor or acceptor sites, or any other site within an exon or intron involved in the definition of splicing, an oligonucleotide can prevent a splicing reaction and cause the exclusion of the targeted exon from a fully-processed mRNA. Exon skipping is achieved in the nucleus during the maturation process of pre-mRNAs. Exon skipping includes the masking of key sequences involved in the splicing of targeted exons by using antisense oligonucleotides that are complementary to such key sequences within a pre-mRNA. For example, the oligomeric compounds provided herein may be suitably employed for exon skipping through the masking of splice sites at intron/exon junctions within a dystrophin pre-mRNA thereby facilitating the deletion of a mutated exon during the processing of the pre-mRNA to a mature mRNA. In the present invention, the oligomeric compound as previously defined are capable of provoking skipping of exon 51 of the human DMD pre-mRNA The expression "provoke skipping of exon 51 of the human DMD pre-mRNA", as used and described in detail herein, refers to the exclusion of exon 51 allowing the rescue of the DMD mRNA reading-frame (e.g., in cells from patients with appropriate mutations), which can be translated into a truncated semi-functional protein.

It is understood in the art that splice-switching strategies can be used for the treatment of patients with DMD disease. In particular, a significant subset of patients with DMD, corresponding to those having large deletions taking away one or several exons such as Δ43-50, Δ45-50, Δ47-50, Δ48-50, Δ49-50, Δ50, Δ52 or Δ52-58, could potentially benefit from the present invention aiming to realize skipping of exon 51, although clinical benefit for each patient will depend on the quality of the truncated dystrophin generated from his specific genetic deletion.

Those skilled in the art will recognize that there are many ways to determine or measure a level of efficacy in response to a treatment such as splice switching. Such methods include but are not limited to measuring or detecting an activity of the rescued protein in patient cells or in appropriate animal models. It is also possible to gauge the efficacy of a treatment protocol intended to modify the exon composition of a mRNA by using RT-PCR for assessing the presence of the targeted exon in patient cells as well as in normal cells or in wild type animal models if interspecies homology permits.

Other features and advantages of the present invention will become apparent from the following detailed description which makes reference to the accompanying drawings.

### BRIEF DESCRIPTION OF THE DRAWINGS

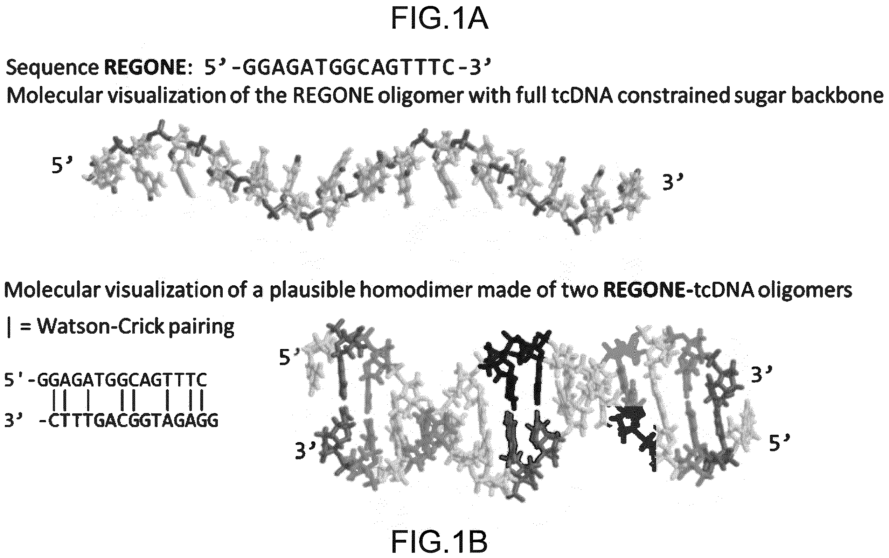

Figure 1 illustrates the sequence REGONE (SEQ ID NO:3). Figure 1A shows the very shape of the REGONE sequence with full tc-DNA constrained sugar backbone - 3D modeling was achieved by using a set of appropriate computer tools and published experimental data obtained from NMR, CD spectroscopic structural investigations and crystal structure of tc-DNA. Figure 1B shows the partial pairing of the REGONE sequence with itself - The symbol "|" refers to possible Watson-Crick pairing - If it were an oligomer made up of canonical DNA, this structure would be unstable (Tm below 15°C). However, 3D modeling of these dimers suggests that such forms may exist in the presence of a preorganized constrained backbone such as tc-DNA - "GΞC" pairings are indicated in black, "A=T" parings are in dark grey, nucleotides not involved in base pairing are in soft grey.

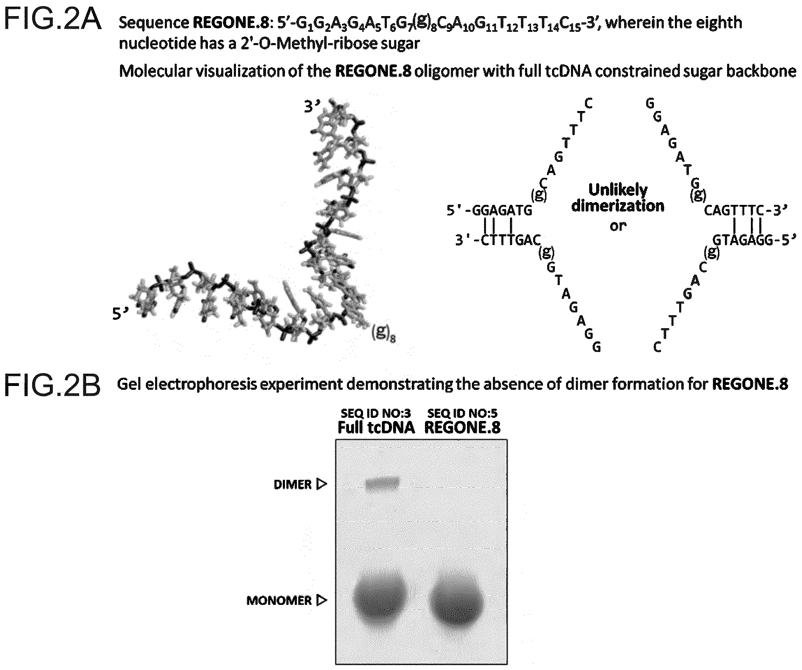

Figure 2 illustrates the outcome on the overall shape of the REGONE tc-DNA-based oligonucleotide after changing a single tc-DNA nucleotide by an equivalent nucleotide with 2OMe-ribose sugar. Figure 2A shows the putative effect of such modification at position 8 on the 3D shape - Subsequent oligomer is named "REGONE.8" (SEQ ID NO:5). The 2'OMe-nucleotide introduces a point of flexion within the tc-DNA string, which disrupts its overall preorganized structure, thus abolishing its capacity to sustain dimeric forms raising from incomplete base-pairing. Figure 2B shows gel electrophoresis experiments in non-denaturing conditions demonstrating that REGONE.8 is indeed no longer able to form dimers. The same sequence consisting only of tcDNA nucleotides systematically presents dimerized forms migrating differently in the gel (note that the proportion of dimerized forms may be higher).

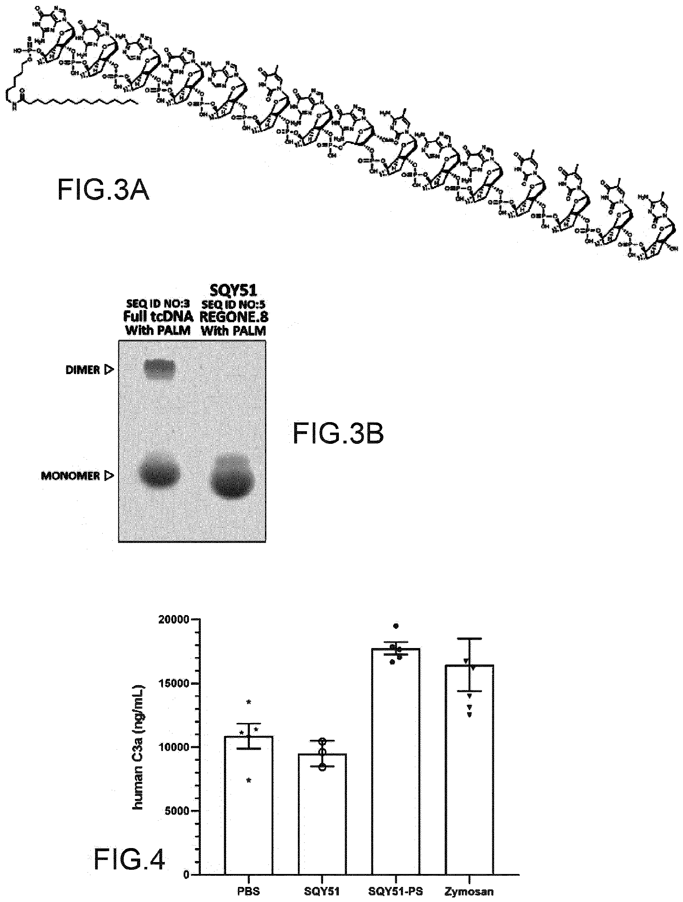

Figure 3 illustrates the composition of the compound SQY51. Figure 3A shows that it comprises the antisense oligonucleotide REGONE.8 (SEQ ID NO:5) covalently attached to a palmitoyl residue at its 5' end via a C6 linker. The Chemical Formula of SQY51 is C₂₁₅H₂₆₃N₆₀O₉₃P₁₅S; Exact Mass 5669.35; Molecular Weight 5672.46. Figure 3B shows gel electrophoresis experiments in non-denaturing conditions demonstrating that the addition of the palmitoyl residue has no effect on the dimerization properties of the oligonucleotides. SQY51 is still not able to form dimers while its counterpart with full tc-DNA nucleotides does.
Figure 4 illustrates the results of a complement assay using human serum for SQY51 and the same compound with full phosphorothioate internucleoside linkages (SQY51-PS). Phosphate buffered saline (PBS) was used as negative control for complement activation, whereas Zymosan, a glucan with repeating glucose units connected by β-1,3-glycosidic linkages found on the surface of yeast, was used as positive control. The experimental drug concentration for *in vitro* testing of complement activation was 2 mg/mL, which mimics an *in vivo* dose-regimen of about 150 mg/kg with a theoretical Cₘₐₓ of about 0.4 mM (likely much more since we considered the volume of blood as plasma in this extrapolation).

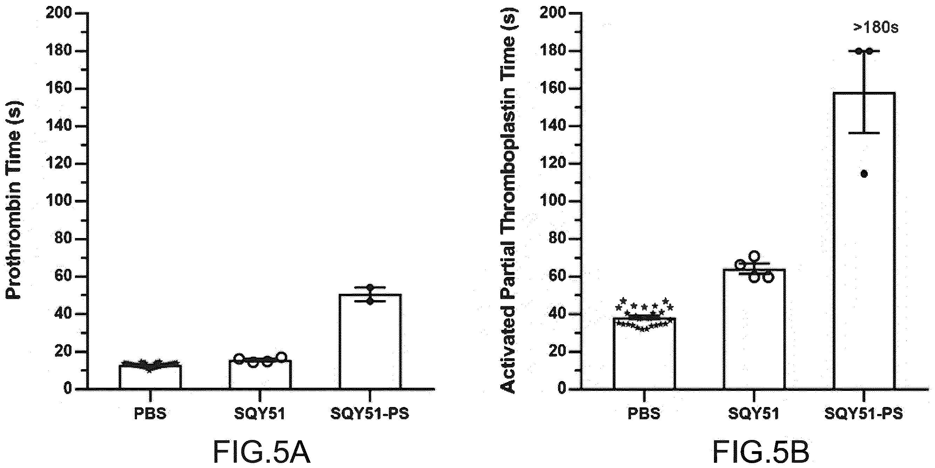

Figure 5 illustrates the results of clotting assays using human plasma for SQY51 and the same compound with full phosphorothioate internucleoside linkages (SQY51-PS). Phosphate buffered saline (PBS) was used as control. The Prothrombin Time (PT, Figure 5A) and the Activated Partial Thromboplastin Time (APTT, Figure 5B) are two blood-tests that measure how long it takes for blood to clot in the presence of an experimental drug.

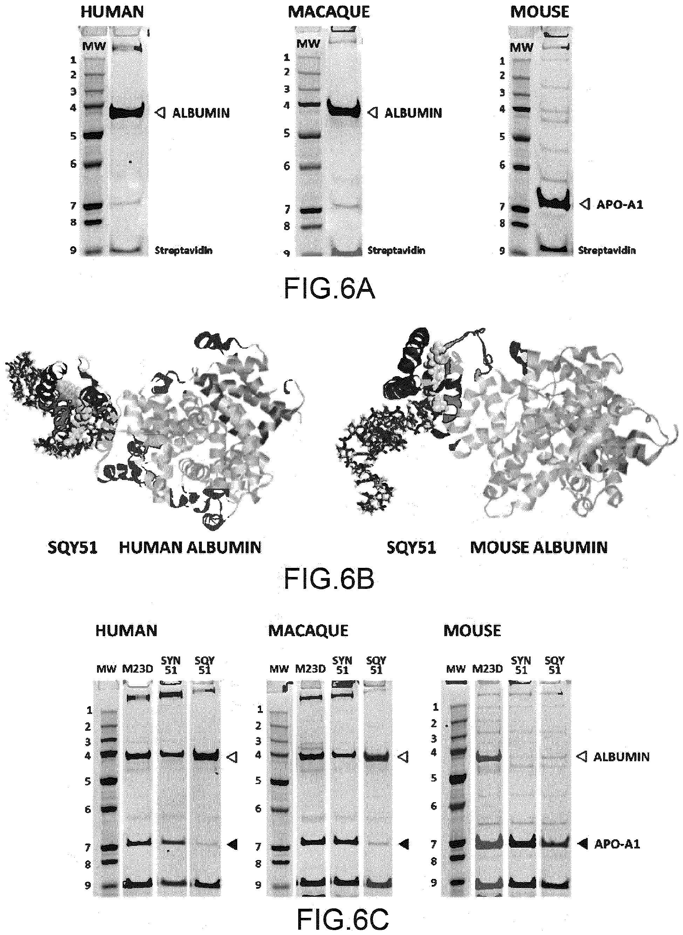

Figure 6 illustrates: Figure 6A: SDS-PAGE analysis of protein recovery from human, macaque and mouse sera using a biotinylated-SQY51 molecule (the biotin moiety was attached at the 3' end of the oligonucleotide depicted in FIG. 3 through a C3 linker - not shown). Figure 6B: 3D-modeling of human albumin and mouse albumin interacting with SQY51. Albumin is represented as "ribbons", the palmitoyl-C6-amino in "real volume", and REGONE.8 as "batons". Figure 6C: SDS-PAGE analysis of protein recovery from Human, macaque and mouse sera using different biotinylated compounds such as SYN51 (Palmitoyl-amino-C6-SEQ ID NO: 8), M23D (Palmitoyl-amino-C6-SEQ ID NO: 9), and SQY51 (Palmitoyl-amino-C6-SEQ ID NO: 5); a biotin moiety was attached at the 3' end of each oligonucleotide through a C3 linker.

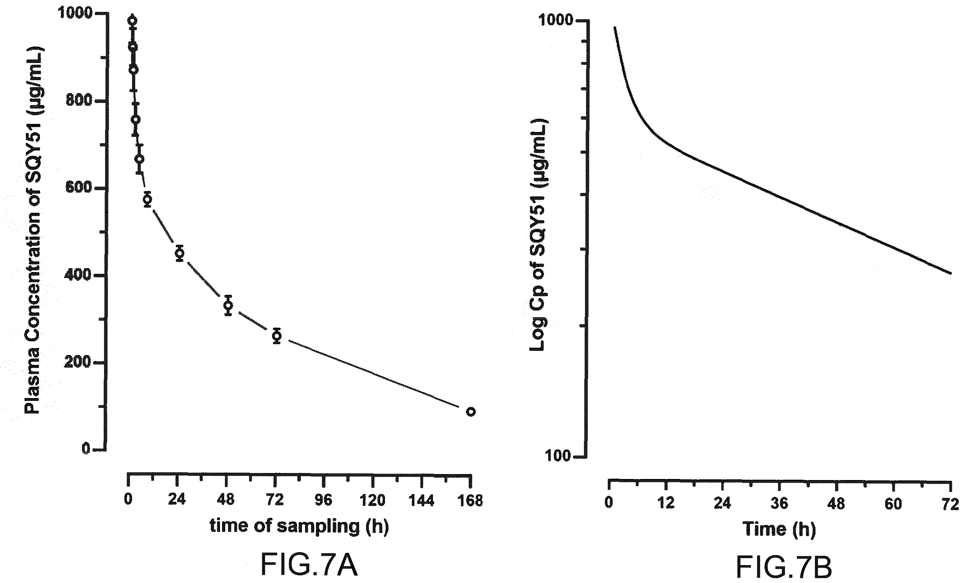

Figure 7 illustrates determination of blood pharmacokinetic (PK) parameters of SQY51 after intravenous infusion (dose of 50 mg/kg - 30 min infusion) in cynomolgus monkey (*Macaca fascicularis*)*.* Typically, determination of PK parameters requires a number of blood samples taken at different time points to estimate maximum concentration in plasma (Cmax), half-life (t½), volume of distribution (VD) and area under the curve (AUC). Figure 7A shows plasma concentration of SQY51 over a week after infusion. Figure 7B shows time profile deduced from a two-compartment model (Phoenix WinNonlin 8.1). Plasma concentration (Cp) of SQY51 follows a bi-exponential kinetics as previously described for other sorts of oligonucleotides. PK parameters for SQY51 are: Cmax = 900 ± 120 µg/mL; αt½ = 2 ± 0.3 h; βt½ = 90 ± 3 h.

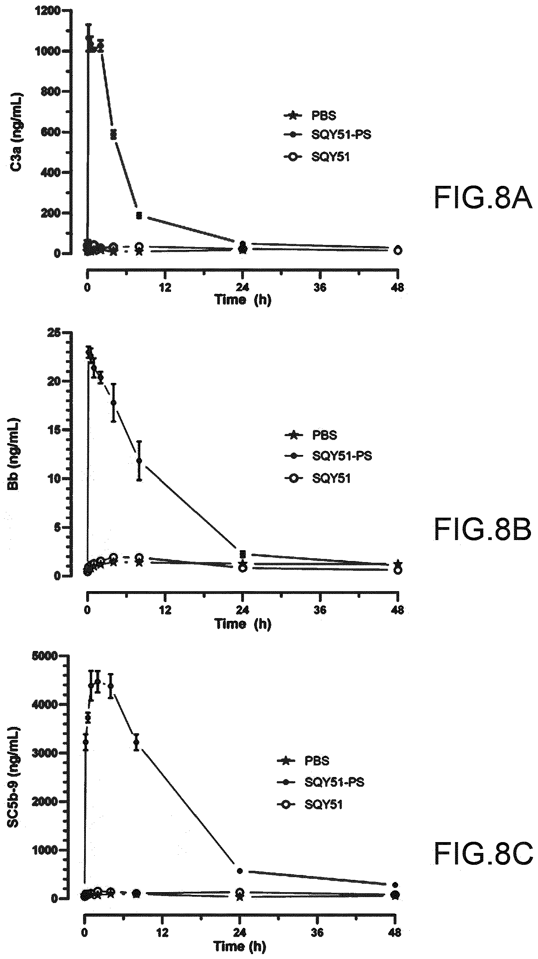

Figure 8 illustrates the results of complement activation in cynomolgus monkey (*Macaca fascicularis*) at different time points (pretest, 10', 30', 1h, 2h, 4h, 8h, 24h and 48h) after systemic delivery of SQY51 via an intravenous infusion using a dose of 50 mg/kg in phosphate buffered saline (PBS). PBS alone and a corresponding compound with full phosphorothioate internucleoside linkages (SQY51-PS) were used as negative and positive controls, respectively. Figure 8A shows the progression of C3a levels, which mirror the C3 consumption. Figure 8B shows the progression of Bb, a proteolytic enzyme, which is generated by the cleavage of the factor B during the activation of the alternative pathway (AP) of the complement system. Figure 8C shows the development of SC5b-9, a soluble complex of S Protein and C5b-9 resulting from the activation of complement in the absence of bilipid layer membranes.

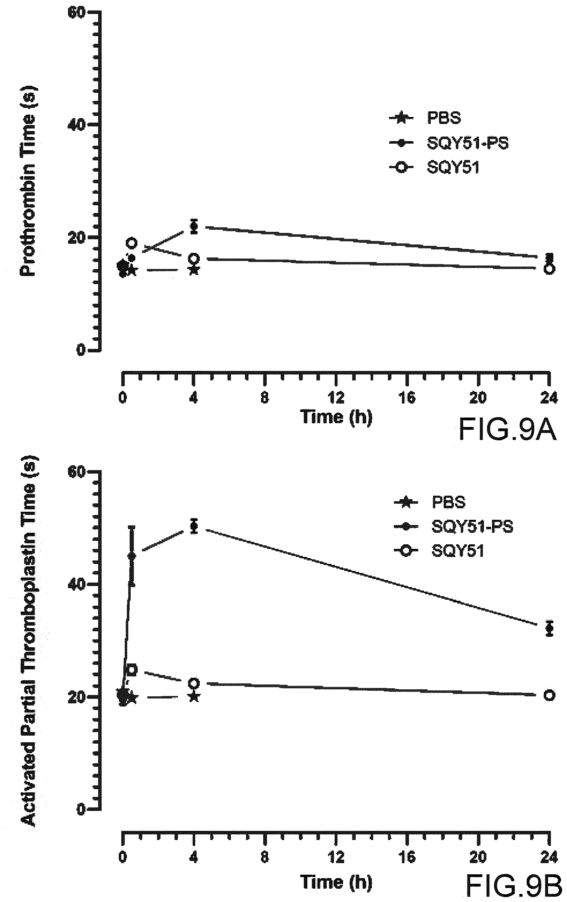

Figure 9 illustrates the results of clotting assays in blood samples from cynomolgus monkey (*Macaca fascicularis*) at different time points (pretest, 30', 4h and 24h) after systemic delivery of SQY51 via an intravenous infusion using a dose of 50 mg/kg in phosphate buffered saline (PBS). PBS alone and a corresponding compound with full phosphorothioate internucleoside linkages (SQY51-PS) were used as negative and positive controls, respectively. The Prothrombin Time (PT, Figure 9A) and the Activated Partial Thromboplastin Time (APTT, Figure 9B) are two blood-tests that measure how long it takes for blood to clot in the presence of an experimental drug.

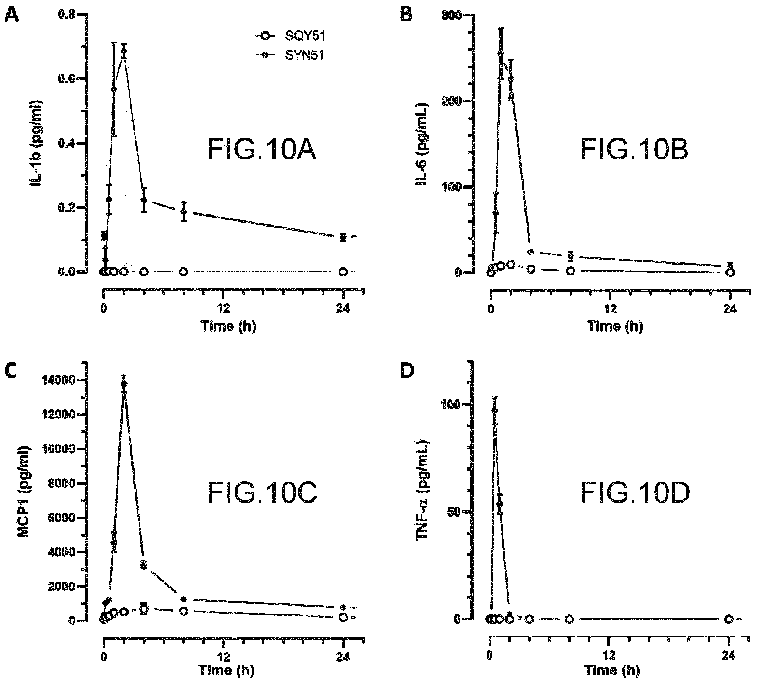

Figure 10 illustrates cytokines levels in blood samples over 48h post-infusion after a single dose of SQY51 (50 mg/kg - 30 min infusion). The results obtained with SYN51-PS are superimposed to exemplify the consequences of a compound triggering a toxic response. Are disclosed the levels of Interleukin 1 beta (IL-1β) (Figure 10A), the levels of Interleukin 6 (IL-6) (Figure 10B), the levels of Monocyte Chemoattractant Protein 1 (MCP-1) (Figure 10C) and the levels of Tumor Necrosis Factor alfa (TNF-α) (Figure 10D).

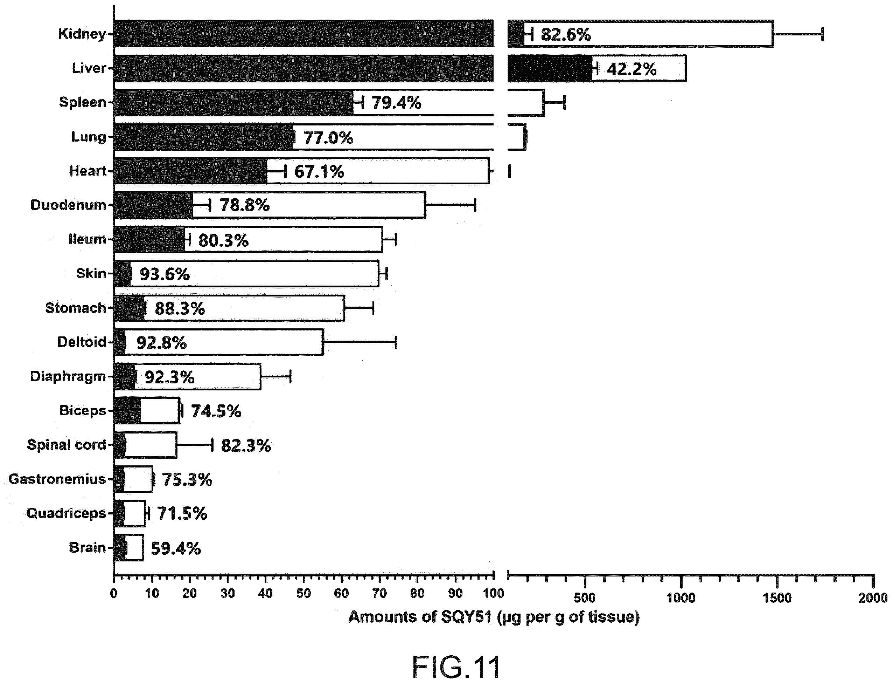

Figure 11 illustrates biodistribution of SQY51 in monkey tissues after one week or five weeks after the end of a 4-week treatment with a dosage of 50 mg/kg/week; amounts of compounds were measured with the beacon method. Graph shows superimposed results for SQY51 quantification at one week after the end of the treatment (white plots) and after 4 additional weeks (black plots). The % indicate the rate of clearance after 4 weeks of washout.

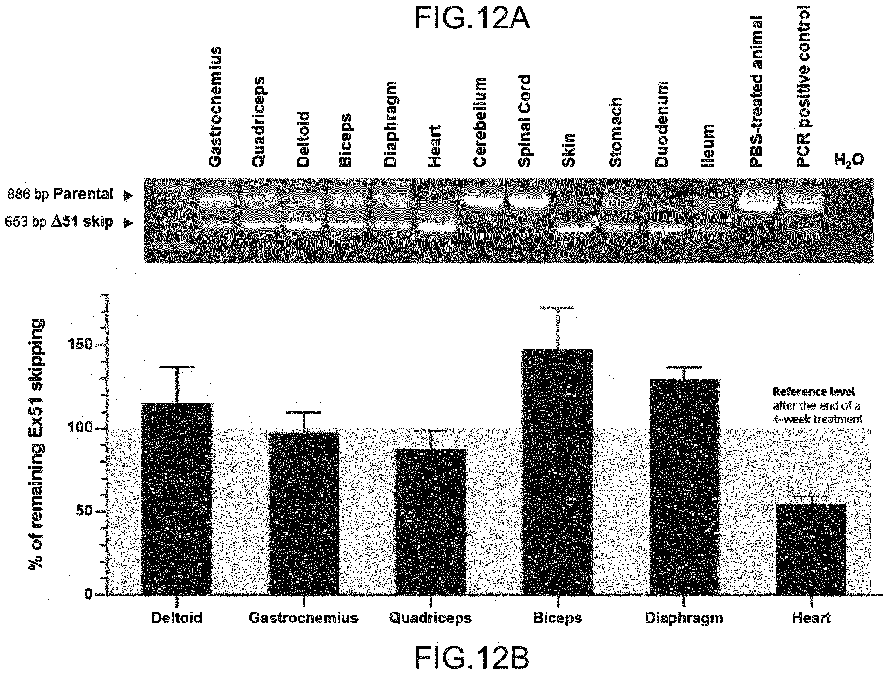

Figure 12 illustrates levels of exon 51-skipped dystrophin mRNA in monkey tissues. Figure 12A exemplifies detection of exon-51 skipping using nested RT-PCR in gastrocnemius, quadriceps, deltoid, biceps, diaphragm, heart, cerebellum, spinal cord, skin, stomach, duodenum and ileum, one week after 4 weekly injections of SQY51 with a dosage of 50 mg/kg/week. Reverse-transcribed mRNAs were sequentially amplified with PCR1 (Ex46F/Ex53R) and PCR2 (Ex47Fi/Ex52Ri) in order to picture an amplicon of 886 base-pairs (bp) rising from normally spliced dystrophin mRNAs, and an amplicon of 653 bp missing exon-51 as a result of the effect of SQY51. Figure 12B shows the percentage of remaining skipping levels in striated muscles at 5 weeks post-treatment (black plots) comparatively to levels at one week after last injection (the background display in grey refers to the levels attained at one-week post-treatment for each muscle; n=4 animals per group).

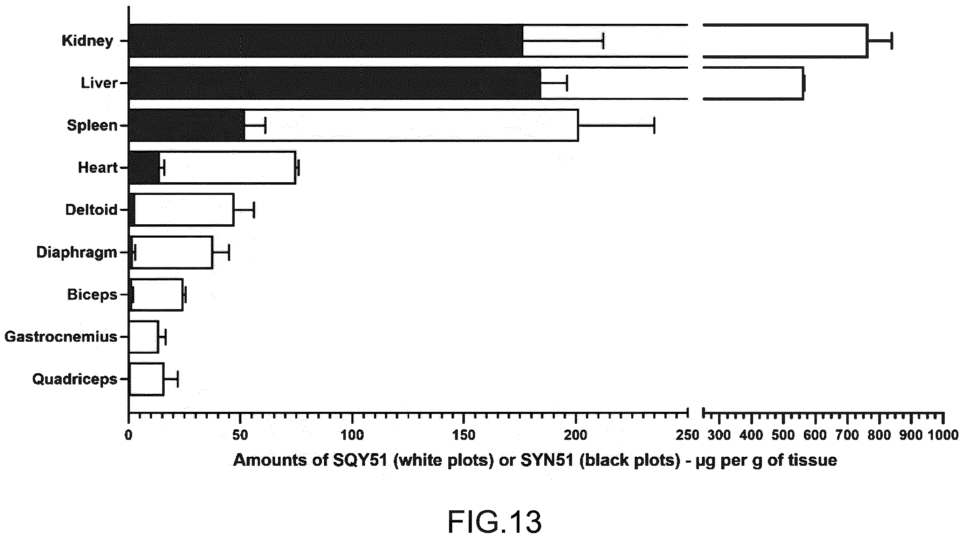

Figure 13 illustrates comparison between biodistribution of SQY51 and SYN51 in monkey tissues one week after a 4-week treatment with a dosage of 50 mg/kg/week; amounts of compounds were measured using LC-MS/MS. Graph shows superimposed results for SQY51 quantification (white plots) and SYN51 (black plots).

### DETAILED DISCUSSION OF PARTICULAR EMBODIMENTS

### Example 1: Compounds for the treatment of Duchenne muscular dystrophy

Typically, small antisense oligonucleotides involve the use of nucleic acids whose skeleton is made more rigid by means of a constrained sugar backbone, as it is the case for LNAs (locked nucleic acids) or tricyclo-DNA as examples. Figure 1 illustrates the REGONE sequence (SEQ ID NO:3) made of tricyclo-DNA nucleotides, whose constrains on ribose-moieties impose an overall shape to the oligomer so that its Tm is increased by 2-3°C per nucleotide. As a reminder, the melting temperature (Tm) of an oligonucleotide or oligomeric compound refers to the temperature at which 50% of the oligonucleotide is in a duplex with its complement. The counterpart of this preconfigured rigidity is that a sequence not supposed to form homodimers could still do so and therefore prove to be highly toxic in vivo, in particular when the internucleotide links are of phosphorothioate (PS) type to improve biodistribution **[6,10].**

The tendency of REGONE to homodimerize was solved by introducing an unconstrained nucleotide within the sequence. As illustrated in Figure 2, such modification upsets the very organization of the tricyclo-DNA oligomer, which has now an inner degree of flexibility so that an imperfect pairing will not be maintained. This is confirmed by (i) 3D-modeling of a REGONE variant where the eighth tc-DNA nucleotide is replaced by an equivalent 2'-*O*-Methyl-ribose RNA (so called REGONE.8), and (ii) in gel electrophoresis experiments demonstrating that subsequent compound is no longer able to form homodimers. Maximal effect was obtained for modification at nucleotide number-8 and -9, to less extent at nucleotide number-7 and -10, and not at all at other positions. Thus the modification should be located between nucleotides 7 and 10 - REGONE.7 (SEQ ID NO: 4), REGONE.8 (SEQ ID NO: 5), REGONE.9 (SEQ ID NO: 6) and REGONE.10 (SEQ ID NO: 7). Other modifications - at position 1, 2, 3, 4, 5, 6, 11, 12, 13, 14 or 15 - do not satisfactorily affect the overall preorganized structure of the molecule, which remains capable to tolerate dimer formation.

In order to guarantee, or at least not compromise, the safety of REGONE.8 under intravenous injection conditions, it was preferred to link the 15 nucleotides of the oligomer by phosphodiester (PO) bonds and not phosphorothioate (PS) like those skilled in the art would advocate to satisfy an effective biodistribution. Furthermore, the oligomers of the tricyclo-DNA class are satisfactorily stable in biological fluids and therefore do not require this modification (i.e., PS-bonds) which, on the contrary, could prove to be disadvantageous if the compound were no longer biodegradable. Finally, the REGONE.8 (SEQ ID NO: 5) was covalently attached to a palmitoyl residue at its 5' end via a C6 linker as shown in Figure 3. This ultimate compound (C₂₁₅H₂₆₃N₆₀O₉₃P₁₅S) is named SQY51.

### Example 2: In vitro toxicity assessment of the invention on human blood

Advantageously, SQY51 does not activate complement in human serum, while the same compound with phosphorothioate internucleoside linkages (SQY51-PS) does (Figure 4); especially considering that the experimental drug concentration for such in vitro assay was 2 mg/mL, which mimics an in vivo dose-regimen of about 150 mg/kg with a theoretical Cₘₐₓ of about 0.4 mM - likely much more as we considered the volume of blood as plasma for this extrapolation.

Another feared problem during intravenous injections of AONs is their possible interaction with the coagulation system. Several routine tests exist to assess the effect of candidate drugs on coagulation such as Prothrombin Time (PT) and Activated Partial Thromboplastin Time (APTT). The latter are two blood-tests that measure how long it takes for blood to clot in the presence of an experimental drug). Prolongation of clotting times (PT & APTT) indicates an anticoagulant effect (risk of excessive bleeding), while shortening indicates a procoagulant effect (risk of developing clots - thrombosis). The experimental drug concentration for clotting assays was 2 mg/mL, which mimics an *in vivo* dose-regimen of about 150 mg/kg with a theoretical Cₘₐₓ of about 0.4 mM (likely much more since we considered the volume of blood as plasma in this extrapolation). Figure 5 clearly shows that SQY51 does not significantly modify clotting parameters in human plasma, while SQY51-PS does, particularly by increasing the clotting time of the extrinsic coagulation pathway (APTT).

### Example 3: Oligo-captured serum proteins of the invention

A further important subject is which experimental model to use to validate an innovation in the field of AONs. Most often, the skilled person merely established the efficacy of a novel AON on appropriate cells in tissue culture then perhaps *in vivo* using murine models. Unfortunately, such approach presupposes that the antisense compound will interact with the body fluids in the same way regardless of the species, a postulate which must be confirmed. To this end, we investigated which proteins in the serum were capable of binding to SQY51. While most of the sequences tested (i.e., with same type of design) gave very similar capture profiles, it is remarkable that SQY51 is unique because it presents major differences between species.

SQY51 molecule preferentially retains serum albumin in the human and the macaque sera whereas it is APO-A1 in the mouse serum (Figure 6A).

Molecular dynamic studies with GROMACS **[11]** indicate that SQY51 can interact with the mouse albumin only through its palmitoyl moiety (Figure 6B). In agreement with experimental data shown in Figure 6A, the 3D-analysis shows that SQY51 interacts more strongly with the human albumin; here, in addition to the interaction with the palmitoyl residue, the REGONE.8 moiety can also interact with the protein to strengthen the assembly.

Figure 6C discloses the SDS-PAGE analysis of protein recovery from human, macaque and mouse sera using different biotinylated compounds such as SYN51 (Palmitoyl-amino-C6-SEQ ID NO: 8), M23D (Palmitoyl-amino-C6-SEQ ID NO: 9), and SQY51 (Palmitoyl-amino-C6-SEQ ID NO: 5); a biotin moiety was attached at the 3' end of each oligonucleotide through a C3 linker. The sequence SEQ ID NO: 8 in the appended sequence listing reads: 5'-AAGAuGGCATTTCTA-3' and the sequence SEQ ID NO: 9 in the appended sequence listing reads: 5'-CCTCGGCTTACCT-3' in which tcDNA nucleosides are typed in capital letters while the 2'OMe-nucleoside is typed in lowercase letter.

Note that M23D is a full tc-DNA oligomer, while SYN51 and SQY51 both comprise a within the tc-DNA chain. M23D captured a similar pattern of proteins independently of tested species (albumin and apolipoproteins), which is similar to that of SYN51 in Human and macaque. Only SQY51 has the characteristic of preferentially fixing albumin in primate sera whether of human or non-human origin.

As shown in Figure 6, SQY51 preferentially retains serum albumin in human and non-human primate sera while it interacts predominantly with apolipoproteins in mouse. 3D-modeling of SQY51 with human and mouse albumin confirmed that both proteins could take over the palmitoyl residue. Nonetheless, it appeared also that the REGONE.8 moiety of SQY51 could itself interact with the human albumin to strengthen the assembly, something that does not take place with the mouse albumin. This species-specific performance of the SQY51 is unique and was obviously not foreseeable by those skilled in the art. It also indicates that it is essential to further assess the advantages of this compound in a suitable animal model (e.g., non-human primate) such as cynomolgus monkey.

### Example 4: Pharmacokinetics and evaluation of toxicity of the compounds according to the invention in cynomolgus monkey

First of all, the inventors compared pharmacokinetics of SQY51 to those of SYN51, also comprising an oligomeric 15-mer sequence having tricyclo-DNA nucleosides and a 2'-O-modified-RNA nucleoside (SEQ ID NO: 8) and a lipid moiety.

It is noteworthy in Figure 7A that SQY51 is stable in the blood compartment (e.g., mass spectrometry analysis reveals that SQY51 remained mostly intact in plasma samples throughout the kinetic study - not shown - although a minor form, lacking the palmitoyl residue and culminating at about 0.1% of Cmax, could be detected at 24h after infusion).

Importantly, contrasting with SQY51, serum-protein capture by SYN51 showed parallel set of proteins regardless of the species (e.g., serum albumin and apolipoproteins). This difference in affinity for blood proteins was reflected in pharmacokinetic profiles. Indeed, although the two compounds follow a bi-exponential decay after intravenous infusion (50 mg/kg), their secondary PK parameters in blood are different; a 2-compartmental analysis (2C model) gives an elimination half-life of about 20 hours for SYN51 while it is about 5 times longer for SQY51 (Figure 7).

The below Table 1 shows comparison of secondary PK parameters for SQY51 and SYN51 after a single (50 mg/kg) intravenous infusion in cynomolgus monkeys. Amounts of SQY51 and SYN51 in blood samples at different time points were assessed by LC-MS/MS. Secondary PK parameters have been calculated from 2C model (bi-exponential kinetics).

**TABLE 1**

| Secondary PK Parameters | SQY51 | | SYN51 | |
|---|---|---|---|---|
| | Mean | CV% | Mean | CV% |
| α t½ (h) | 2.01 | 21.55 | 0.70 | 8.10 |
| β t½ (h) | 90.14 | 38.99 | 21.74 | 0.92 |
| AUC (µg/mL*h) | 58609.60 | 25.73 | 19406.10 | 9.15 |
| Cmax (µg/mL) | 880.76 | 0.62 | 1018.76 | 7.35 |
| MRT (h) | 127.04 | 38.77 | 30.65 | 0.61 |
| VSS | 106.49 | 13.73 | 79.32 | 9.76 |

It is important to note that SQY51 remained stable throughout the elimination phase - Mass spectrometry analyzes all over the study revealed that SQY51 remained intact in the blood flow and retained its palmitoyl moiety.

Given the high persistence of SQY51 in the blood, due to its special binding properties with primate-serum albumin, it was central to check whether such prolonged presence would trigger deleterious events such as complement activation (Figure 8), coagulation misfunctioning (Figure 9), and rising of pro-inflammatory cytokines (Figure 10). None of these potentially harmful events were observed with SQY51, but they were saw when the compound contained PS-type internucleotide bonds.

Concerning the pro-inflammatory cytokines studied in Figure 10, the interleukin 1 beta (IL-1β) is produced by activated macrophages and it is an important mediator of the inflammatory innate response. The interleukin 6 (IL-6), an inflammatory cytokine, which can be secreted by macrophages in response to molecules referred to as pathogen-associated molecular patterns (PAMPs) that bind to detection molecules of the innate immune system, called pattern recognition receptors (PRRs), including Toll-like receptors (TLRs), that are present on the cell surface and intracellular compartments. Monocytes/macrophages are the major source of Monocyte Chemoattractant Protein 1 (MCP-1), although it can be produced by other cell types, including endothelial cells and fibroblasts, which are important for antiviral immune responses in the peripheral circulation and in tissues. Tumor Necrosis Factor alfa (TNF-α) is a pro-inflammatory cytokine naturally produced by activated macrophages and monocytes in response to infection and injury, which mediates hypotension, diffuse coagulation, and widespread tissue damage.

Analysis of the biodistribution of SQY51 in the various tissues of the body was carried out at one week and five weeks after four weekly-injections at a dose of 50 mg/kg (Figure 11). First, it appears as expected that the kidney and liver are the main organs that accumulate the compound.

The below Table 2 shows comparison of exon-51 skipping in cynomolgus muscles one week after a 4-week treatment (50 mg/kg/week) with either SQY51 or SYN51. On average, SQY51 is 10 times more efficient than SYN51.

**TABLE 2**

| **% exon-51 skipping in striated muscles** | **SQY51 Mean** | **SYN51 Mean** |
|---|---|---|
| Deltoid | 27.1 | 0.0 |
| Gastrocnemius | 16.3 | 1.3 |
| Quadriceps | 17.2 | 1.6 |
| Biceps | 16.8 | 1.9 |
| Diaphragm | 18.9 | 2.8 |
| Heart | 36.8 | 5.5 |
| AVERAGE | 22.2 | 2.2 |

Thus, among the striated muscles, the heart seems to be a prime target. Second, it is important to note that the amount of SQY51 decreased very significantly in tissues after only 4 additional weeks of wash-out, particularly in kidney. Indeed, we observed a clearance of over 70% in all tissues after four weeks post-treatment.

### Example 5: Efficacy of the compound according to the invention in cynomolgus monkey

Nested RT-PCR analysis showed levels of exon-51 skipping in monkey tissues after systematic administration of SQY51 (Figure 12), thus confirming widespread delivery of its antisense moiety to myo-nuclei of the whole musculature, including skeletal muscles, heart and smooth muscles.

Noteworthy, despite the high clearance rate of SQY51 in muscles (see Figure 11), levels of exon 51 skipping at 5 weeks were relatively similar to those detected at 1 week, and thus are almost maintained regardless of the wash-out, indicating that the cleared compound was mostly an unproductive fraction of SQY51 possibly stranded in interstitial fluids or in endosomal compartments where it is cleared out or underwent destruction. Furthermore, this lasting effect indicates that treatment with SQY51 can be discontinued for periods longer than one or two months without significant loss of benefit, thus offering the body the time to get rid of putative excess of the tricyclo-DNA oligomer in tissues at risk such as the kidney and liver.

Finally, the novel compound SQY51 has crucial advantages over SYN51, which was so far considered as the finest tc-DNA-based compound for skipping the exon-51 in DMD: (i) because of its inner properties (e.g., higher specific binding to human & NHP serum albumin) SQY51 shows improved biodistribution in monkey muscles after systemic delivery (Figure 13), and (ii) subsequent exon-51 skipping levels in striated muscles were 10 times higher, prejudging that SQY51 has a real therapeutic interest.

### REFERENCES

**[1]** Lu et al, Mol. Ther. Nucleic Acids. 2014, vol. 3, e152.
**[2]** International application WO 2010/115993 published on October 14, 2010.
**[3]** International application WO 2013/053928 published on April 18, 2013.
**[4]** Goyenvalle et al, Nat. Med. 2015, vol. 21, pages 270-275.
**[5]** Dirin and Winkler, Expert Opin. Biol. Ther. 2013, vol. 13, pages 875-888.
**[6]** Echevarria et al, Nucleic Acid Ther. 2019, vol. 99, pages 148-160.
**[7]** Iwamoto et al, Nat. Biotech. 2017, vol. 35, pages 845-851.
**[8]** International application WO 2018/193428 published on October 25, 2018.
**[9]** Bell et al, ChemBioChem, 2009, vol. 10, pages 2691-2703.
**[10]** Aupy et al, Mol Ther Nucleic Acids. 2019, vol. 19, pages 371-383.
**[11]** Abraham et al, SoftwareX, 2015, vol. 1-2, pages 19-25.

## Claims

1. Oligomeric compound comprising from 10 to 50 monomer subunits, at least part of the sequence of which is complementary to the following sequence: AAGGAAACUGCCAUCUCCAA (SEQ ID NO: 1 in the appended sequence listing).

2. Oligomeric compound according to claim 1, wherein at least part of the sequence of said oligomeric compound is complementary to the sequence corresponding to the region +48+62 of SEQ ID NO: 2 in the appended sequence listing.

3. Oligomeric compound according to claim 1 or 2, wherein it comprises or consists of an antisense oligonucleotide.

4. Oligomeric compound according to any one of claims 1 to 3, wherein it comprises at least one nucleotide sequence having at least 70% identity with the following tc-DNA nucleotide sequence:
GGAGATGGCAGTTTC (SEQ ID NO: 3 in the appended sequence listing).

5. Oligomeric compound according to any one of claims 1 to 4, wherein it comprises or consists of a tricyclo-DNA antisense oligonucleotide.

6. Oligomeric compound according to any one of claims 1 to 5, wherein it comprises or consists of a tricyclo-phosphorothioate DNA antisense oligonucleotide.

7. Oligomeric compound according to any one of claims 1 to 4, wherein it comprises several tricyclo-deoxyribonucleic acid (tc-DNA) nucleosides and at least one modified ribonucleic acid nucleoside.

8. Oligomeric compound according to claim 7, wherein said modified ribonucleic acid nucleoside is a 2'-*O*-methyl RNA nucleoside.

9. Oligomeric compound according to claim 7 or 8, wherein the monomer subunits of said oligomeric compound are joined by phosphodiester internucleoside linkages.

10. Oligomeric compound according to any one of claims 7 to 9, wherein it comprises or consists of a nucleotide sequence corresponding to one of the following nucleotide sequences:
- 5'-GGAGAT**g**GCAGTTTC-3' (SEQ ID NO: 4 in the appended sequence listing),
- 5'-GGAGATG**g**CAGTTTC-3' (SEQ ID NO: 5 in the appended sequence listing),
- 5'-GGAGATGG**c**AGTTTC-3' (SEQ ID NO: 6 in the appended sequence listing), and
- 5'-GGAGATGGC**a**GTTTC-3' (SEQ ID NO: 7 in the appended sequence listing).
in which tcDNA nucleotides are typed in capital letters while the modified ribonucleic acid nucleoside is typed in lowercase letter.

11. Oligomeric compound according to any one of claims 1 to 10, wherein it is combined with one or more lipid moieties.

12. Oligomeric compound according to any one of claims 1 to 10, wherein it is selected from the group consisting of:
- palmitate-NH-C₆alkylene-OP(=S)(OH)-GGAGAT**g**GCAGTTTC-3' (SEQ ID NO: 4 in the appended sequence listing),
- palmitate-NH-C₆alkylene-OP(=S)(OH)-GGAGATG**g**CAGTTTC-3' (SEQ ID NO: 5 in the appended sequence listing),
- palmitate-NH-C₆alkylene-OP(=S)(OH)-GGAGATGG**c**AGTTTC-3' (SEQ ID NO: 6 in the appended sequence listing), and
- palmitate-NH-C₆alkylene-OP(=S)(OH)-GGAGATGGC**a**GTTTC-3' (SEQ ID NO: 7 in the appended sequence listing).

13. Pharmaceutical composition comprising, as an active ingredient, an oligomeric compound according to any one of claims 1 to 12, and a pharmaceutically acceptable vehicle.

14. Oligomeric compound according to any one of claims 1 to 12 or pharmaceutical composition according to claim 13, for use in treating Duchenne Muscular Dystrophy in a patient in need.
